# EUROPEAN PATENT SPECIFICATION

(11) **EP 1 758 624 B1**
(45) Date of publication and mention of the grant of the patent: **21.12.2011**
(21) Application number: 05856212.5
(22) Date of filing: 15.06.2005
(51) Int. Cl.: A61M 3/02

(54) **DEVICE FOR CONTAINING AND DISPENSING TWO-COMPONENT PRODUCTS, PARTICULARLY VAGINAL OR RECTAL WASHES**
VORRICHTUNG ZUR SPEICHERUNG UND AUSGABE VON ZWEIKOMPONENTIGEN PRODUKTEN, INSBESONDERE FÜR SCHEIDEN- ODER REKTALSPÜLUNGEN
DISPOSITIF DESTINE A CONTENIR ET DISTRIBUER DES PRODUITS A DEUX COMPOSANTS, EN PARTICULIER DES SOLUTIONS DE LAVAGE VAGINAL OU RECTAL

(30) Priority: 17.06.2004 IT MO20040148
(43) Date of publication of application: 07.03.2007
(73) Proprietor: LAMEPLAST S.p.A., Frazione Rovereto sul Secchia (IT)
(72) Inventor: FONTANA, Antonio, I-41012 Carpi (IT)
(74) Representative: Brunacci, Marco
(86) International application number: PCT/EP2005/052776
(87) International publication number: WO 2006/066981

(56) References cited:
- FR-A- 2 094 420
- US-A- 3 354 883
- US-A- 4 405 306
- US-A- 4 709 705

## Description

### Technical Field

The present invention relates to a device for containing and dispensing two-component products, particularly vaginal or rectal washes.

### Background Art

Some two-component substances, comprising a solvent and a solute, such as in particular some medical or pharmaceutical substances, such as for example vaginal or rectal washes, have a high chemical activity; in order to keep their chemical characteristics and their pharmaceutical or medical properties unchanged, it is therefore known to package the individual components separately from each other and to mix them only at the time of use.

It is known, for example, to package the two components in two different containers provided with respective closures; at the time of use, the two containers are opened in order to pour the contents of one container into the other and thus mix them.

If the resulting mixture is a wash, in order to apply it, i.e., to irrigate it, inside the body cavity to be cleansed it is known to apply to the mouth of the container, in which the mixture has formed, a cannula that is packaged separately.

Opening the two containers can alter and contaminate the components contained therein, and accidental leaks and spills can occur in transferring the contents of one container into the other.

In order to obviate these drawbacks, it is known to package the two components in two separate tanks, which are formed inside a single container and are mutually separated by removable closure means, by removing which the two tanks are mutually connected in order to mix their contents.

The container is further provided with a dispensing outlet closed by a stopper; after mixing the two components, the stopper is removed in order to associated with the dispensing outlet an applicator, such as for example a cannula, which is packaged separately.

In particular, containers are known which are substantially constituted by a bottle that is extended by a neck, at the top of which there is a dispensing outlet closed by a first stopper; a portion of the walls of the bottle, formed at the base of its neck, is of the elastically deformable type, by being for example concertina shaped.

Such portion separates the container into two tanks: one formed inside the body of the bottle, between the bottom of said bottle and the deformable portion, and one formed inside its neck, between the deformable portion and the dispensing outlet.

The opening that is formed just above the deformable portion and connects the two tanks is closed by a second removable stopper, which continues downward with an appendage whose outside diameter is substantially larger than the minimum inside diameter at the base of the deformable portion.

By squeezing the deformable portion, the second stopper is removed from the opening by way of the interference between its appendage and the minimum diameter at the base of such deformable portion; the two tanks are thus mutually connected, and the content of the tank formed in the neck is transferred into the tank formed inside the body of the bottle, with which it mixes.

Once mixing has occurred, it is necessary to remove the first stopper, the one that closes the dispensing outlet of the bottle, in order to associate with said outlet an applicator, in particular a cannula, which is packaged separately.

These known containers are not free from drawbacks, including the fact that they do not allow regular and complete dispensing of the mixture formed inside them; the second stopper, once removed from the opening that connects the two tanks, in fact remains floating inside them and can become wedged or obstruct the dispensing outlet.

Another drawback of known containers is that they do not ensure the purity of the mixture formed inside them.

Although they allow to mix the two components inside a single bottle without opening the bottle, they still require to open the bottle in order to associate the application cannula with its dispensing outlet, exposing the mixture contained therein to the risk of contaminations.

Another drawback of known containers is that they require performing awkward and laborious operations for opening and associating the application cannula with the dispensing outlet, not to mention the fact that handling the cannula reduces its hygiene and sterility.

Finally, another drawback of known containers is that they require the separate preparation and packaging of the bottle and of the application cannula that can be associated therewith, with consequent waste of material and long and expensive packaging operations.

From patent document US 3,354,883 it is known a disposable syringe for mixing plural medicaments, comprising a nozzle and a bulb containing a first medicament, wherein the nozzle has an enlarged portion which is coupled with a neck portion of the bulb and is provided with a tubular chamber member, in turn defining at its inside a counterbore suitable for accommodating solid tablets or in general a second medicament intended to be mixed with the first medicament contained in the bulb by forcing the nozzle against the bulb.

It is further known, from patent US 4,405,306, a douche product which comprises a bottle filled with a douching liquid, a reservoir in sealing engagement with the bottle and containing a liquid to be mixed with the douching liquid, and a hollow probe attached to the bottle for dispensing the contents of the bottle, wherein the nozzle and the reservoir have cooperating devices so that, when the nozzle is moved relative to the bottle, the reservoir is opened to permit the contents thereof to drain into the bottle.

It is further known, from patent FR 2.094.420, an apparatus for dispensing liquid in a body's cavity, comprising a bottle, having a neck with a releasing nozzle, and a cannula coupled with the nozzle and having an expanded head which defines with the bottle's bottom a chamber filled with a substance, wherein, in the use, the cannula is made to slide along the nozzle so as to open the chamber and thereby allow the substance filling it to be mixed with a liquid contained in the remaining volume of the bottle.

However even all the devices and embodiments known from the above documents for mixing two basic components, and containing and dispensing the final two-component product so obtained, appear to request further improvements, in particular in order to avoid the risk of any inadvertent mixing of the two basic components, and also to improve the conditions in which the final two-component product is dispensed by the device.

### Disclosure of the Invention

The aim of the present invention is to eliminate the abode-mentioned drawbacks of known containers, by providing a device for containing and dispensing two-component products, particularly vaginal or rectal washes, that allows to keep the two components mutually separate until they are used and to mix them only at the time of use inside a single container, without opening the container.

Within this aim, an object of the present invention is to provide a device that allows to protect the product formed therein from external contaminations and to dispense it evenly and completely.

Another object of the present invention is to provide a device that allows to reduce packaging operations, times and costs and to limit the waste of packaging materials. Another object of the present invention is to provide a device that is simple and easy to use and has improved hygiene.

A still further object of the present invention is to provide a structure that is simple, relatively easy to provide in practice, safe in use, effective in operation, and has a relatively low cost.

This aim and these and other objects that will become better apparent hereinafter are achieved by the present device for containing and dispensing two-component products, particularly vaginal or rectal washes, having all the features recited by independent claim 1.

### Brief description of the Drawings

Further characteristics and advantages of the present invention will become better apparent from the following detailed description of a preferred but not exclusive embodiment of a device for containing and dispensing two-component products, particularly vaginal or rectal washes, illustrated by way of non-limiting example in the accompanying drawings, wherein:

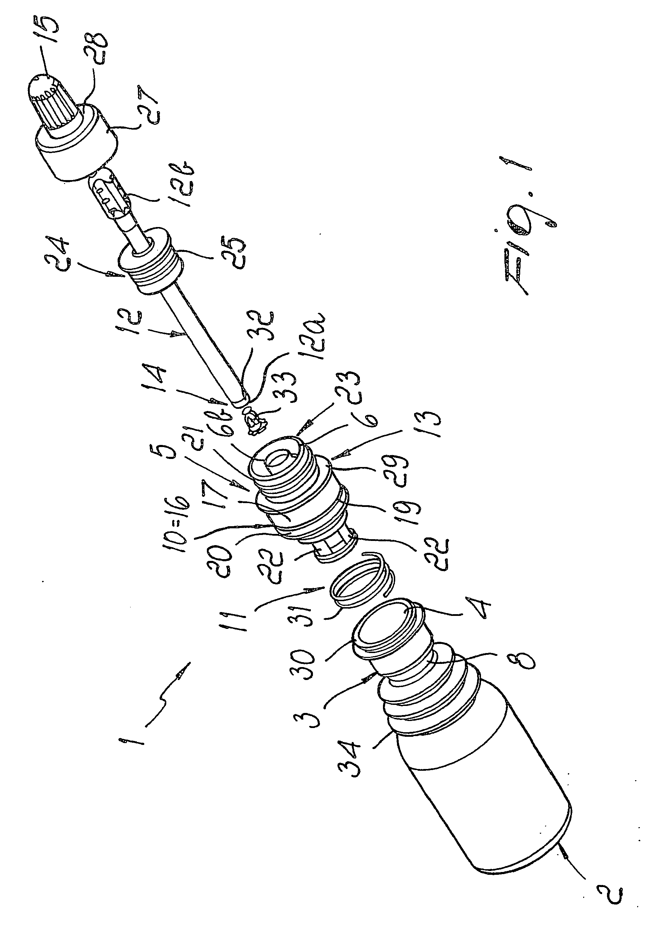

Figure 1 is a schematic exploded view of the device according to the invention;

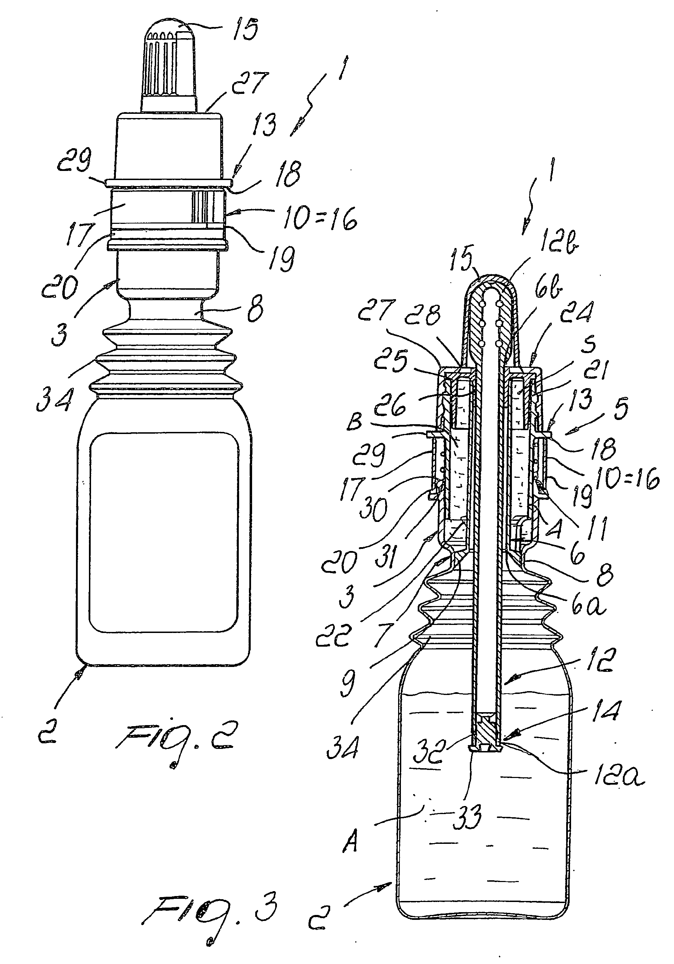

Figure 2 is a schematic side external view of the device according to the invention, in the closed packaging configuration;
Figure 3 is a schematic longitudinal sectional view of the device of Figure 2;

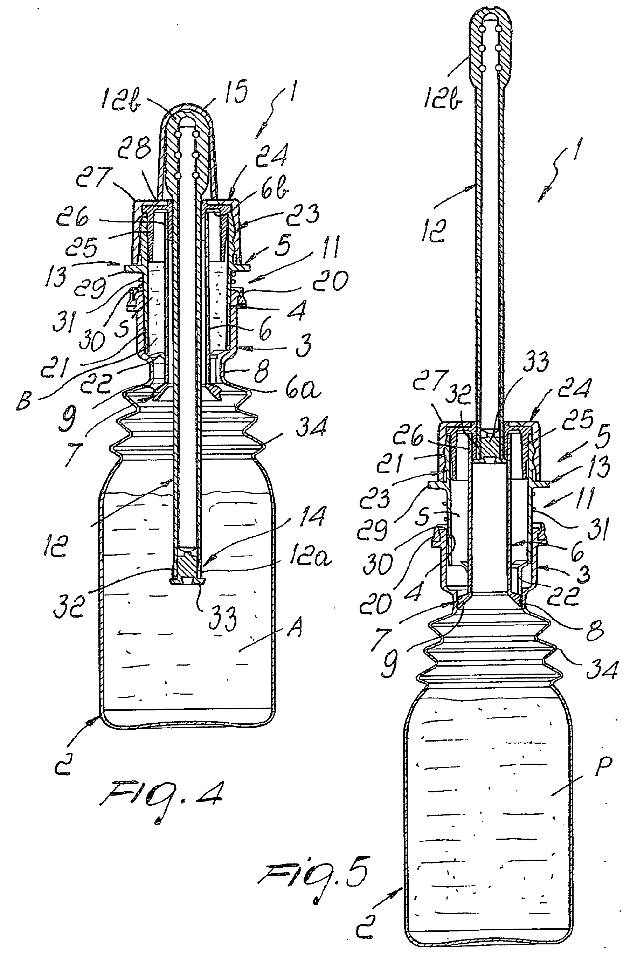

Figure 4 is a schematic longitudinal sectional view of the device according to the invention, in the configuration for mixing the two components;
Figure 5 is a schematic longitudinal sectional view of the device according to the invention, in the configuration for dispensing the mixture of the two components.

### Ways of carrying out the Invention

With reference to the figures, the reference numeral 1 generally designates a device for containing and dispensing two-component products, particularly vaginal or rectal washes.

The device 1 comprises a container 2 of a first component A, which is provided with a neck 3, at the top of which there is an opening 4, and means 5 for closing the opening 4, which are associated, substantially hermetically and so that they can slide axially, with the neck 3 and rigidly support a tubular element 6, the opposite ends 6a and 6b of which are open; said tubular element extends inside the neck 3 substantially coaxially thereto.

The tubular element 6 rigidly supports means 7 for forming a seal with at least one portion 8 of the walls of the neck 3; said means are constituted for example by an annular expansion 9 that protrudes on the outer lateral surface of the tubular element 6 proximate to its end 6a that is directed toward the inside of the container 2.

The volume delimited by the internal surface of the neck 3, by the closure means 5, by the outer lateral surface of the tubular element 6 and by the sealing means 7 forms a tank S for a second component B.

The sliding of the closure means 5 is blocked temporarily by temporary locking means 10, which once released allow the closure means 5 to slide between a first configuration, in which the sealing means 7 interfere with the portion 8, closing the tank S, in order to keep the second component B separated from the first component A, and a second configuration, in which the sealing means 7 are disengaged from the portion 8, opening the tank S in order to pour the second component B into the container 2, where it mixes with the first component A in order to form the product P.

Further, means 11 for returning the closure means 5 to the first configuration are provided.

The tubular element 6 internally accommodates, so that it is substantially coaxial thereto, a tubular dispenser 12, such as for example a cannula, in which the inlet end 12a is directed toward the inside of the container 2 and the outlet end 12b extends outside the tubular element 6.

The dispenser 12 can slide axially and substantially hermetically with respect to at least one portion of the inner lateral surface of the tubular element 6 between a configuration that is substantially retracted toward the inside of the container 2 and a configuration that is substantially extended toward its outside.

The device 1 further comprises first stop means 13 and second stop means 14 for the sliding of, respectively, the closure means 5 and the dispenser 12.

A cap 15 is associated detachably with the outlet end 12b of the dispenser 12.

Between the neck 3 and the closure means 5 there are removable sealing means 16, which conveniently coincide with the temporary locking means 10 and can be constituted for example by an annular band 17, the edges of which are associated, along prefracture lines 18 and 19, respectively with the closure means 5 and with a ring 20, which is coupled with a snap action to the neck 3.

The closure means 5 can comprise, for example, a cylindrical sleeve 21, which is external and substantially coaxial to the tubular element 6 and is associated with the neck 3 so that it can slide axially and substantially hermetically, a plurality of tabs 22 for connecting the sleeve 21 to the tubular element 6 as a single body 23, and a stopper 24 associated with the end of the body 23 that lies opposite the container 2.

The stopper 24 is provided with a first cylindrical appendage 25 for forming a seal with the sleeve 21 and with a second cylindrical appendage 26, which forms a seal with the tubular element 6, and is open at its opposite ends for the passage of the dispenser 12.

A locking ring 27 fixes the stopper 24 to the sleeve 21; conveniently, the cap 15 is associated with the locking ring 27 along a prefracture line 28.

The first stop means 13 comprise a collar 29, which protrudes on the outer lateral surface of the sleeve 21 and a corresponding first annular abutment surface formed by the edge 30 of the neck 3.

The return means 11 can be constituted, for example, by elastic means such as for example a spring 31 interposed between the collar 29 and the edge 30; however, alternative embodiments thereof are not excluded and could be constituted, for example, by a plurality of elastic fins formed below the collar 29, which cooperate with a conical surface formed on the outer lateral surface of the neck 3.

The second stop means 14 can comprise, for example, an annular protrusion 32, which is formed on the outer lateral surface of the dispenser 12 and is suitable to interfere with a corresponding second annular abutment surface formed proximate to the end 6b of the tubular element 6 and in particular in the second appendage 26 and not shown in detail.

A valve 33 is associated with the inlet end 12a of the dispenser 12 and is suitable to prevent the escape of the first component A or of the product P obtained by mixing the first and second components A and B from the container 2 when the dispenser 12 is in the substantially retracted configuration.

The outlet end 12b of the dispenser 12 is constituted by a contoured head, the outside diameter of which is substantially larger than the inside diameter of the tubular element 6 or of the second appendage 26.

Conveniently, a portion 34 of the walls of the container 2, formed at the base of the neck 3, is of the elastically deformable type and can for example be concertina shaped, with its compression axis substantially parallel to the longitudinal axis of the container 2.

The portion 34 allows to tilt the dispenser 12 as needed.

The device 1 is prepared by introducing the first component A in the container 2 and, after arranging the spring 31 so that it rests on the edge 30, by inserting in the neck 3 the body 23 of the closure means 5, which is directly provided with the locking means 10 (which coincide with the sealing means 16) rigidly coupled thereto, until the ring 20 is anchored with a snap action to the neck 3 and the expansion 9 is anchored hermetically to the portion 8.

After inserting the second component B in the tank S, the stopper 23, with the dispenser 12 inserted in the second appendage 26, is applied.

Finally, the locking ring 27, with which the cap 15 is associated temporarily, is applied.

The operation of the invention is as follows.

First of all, it is necessary to eliminate the locking means 10 (which coincide with the sealing means 16) by tearing off the band 17 so as to allow the closure means 5 to slide freely with respect to the neck 3.

By pushing the closure means 5 toward the container 2, overcoming the force of the spring 31, such means are moved from the first to the second configuration, in which the protrusion 9 does not interfere with the portion 8 and opens the tank S, clearing a passage through which the second component B flows out into the container 2, where it mixes with the first component A.

When the thrust force ceases, the spring 31 returns the closure means 5 to the first configuration, closing the tank S again and thus avoiding any reverse flow of the product P into the tank.

Finally, by turning and pulling the cap 15, said cap is disengaged from the locking ring 27 and simultaneously the dispenser 12 is arranged in the extended configuration, ready for the application of the product P.

In practice it has been found that the described invention achieves the intended aim and objects.

The device according to the invention in fact allows to keep the two components mutually separated until the time of use and to mix them only at the time of use inside a single container, without opening said container.

Further, the device according to the invention allows to protect the product formed therein against external contaminations and to dispense it evenly and completely, since no bodies remain floating inside it and no cavities remain in which unusable residues of product can accumulate.

Finally, the device according to the invention allows to reduce packaging operations, times and costs and to limit the waste of packaging materials; the dispenser is in fact integrated therein and is not packaged separately, and this further facilitates and simplifies its use and improves its hygiene.

The invention thus conceived is susceptible of numerous modifications and variations, all of which are within the scope of the appended claims.

All the details may further be replaced with other technically equivalent ones.

In practice, the materials used, as well as the shapes and the dimensions, may be any according to requirements without thereby abandoning the protective scope of the appended claims.

## Claims

1. A device (1) for containing and dispensing two-component products, particularly vaginal or rectal washes, comprising:
a container (2) of a first component (A), which is provided with a neck (3) at the top of which there is an opening (4);
closure means (5) for said opening (4), which are associated, substantially hermetically and so that they can slide axially, with said neck (3) and rigidly support a tubular element (6), which is open at its opposite ends and extends inside said neck (3) and is substantially coaxial thereto;
sealing means (7) for providing a seal with at least one portion (8) of the walls of said neck (3), which are associated with said tubular element (6) or with said closure means (5);
a tubular dispenser (12), which is accommodated in said tubular element (6) and in which the inlet end (12a) is directed toward the inside of said container (2) and the outlet end (12b) extends outside said tubular element (6); and
temporary locking means (10) for locking temporarily the sliding of said closure means (5); **characterized in that**
the volume delimited by the internal surface of said neck (3), by said closure means (5), by the outer lateral surface of said tubular element (6) and by said sealing means (7) forms a tank (S) for a second component (B), and
following the elimination of said temporary locking means (10), said closure means (5) are able to slide between a first configuration, in which the sealing means (7) interfere with said portion (8) of the walls of said neck (3), closing said tank (S), and a second configuration, in which the sealing means (7) are disengaged from said portion, opening said tank (S) in order to pour said second component (B) into said container (2).

2. The device according to claim 1, **characterized in that** said dispenser (12) is accommodated in said tubular element (6) so that it is substantially coaxial and can slide axially with respect to it, substantially hermetically with at least one portion of its inner lateral surface, between a configuration in which it is substantially retracted toward the inside of said container (2) and a configuration in which it is substantially extended toward the outside of said container (2).

3. The device according to one or more of the preceding claims, **characterized in that** it comprises first (13) and/or second means (14) for stopping the sliding respectively of said closure means (5) and/or of said dispenser (12).

4. The device according to one or more of the preceding claims, **characterized in that** it comprises return means (11) for returning said closure means (5) to said first configuration.

5. The device according to one or more of the preceding claims, **characterized in that** it comprises a removable cap (15) for covering said outlet end (12b) of the dispenser (12).

6. The device according to one or more of the preceding claims, **characterized in that** it comprises removable sealing means (16) formed between said closure means (5) and said neck (3).

7. The device according to claim 6, **characterized in that** said temporary locking means (10) and said removable sealing means (16) coincide with, and comprise, an annular band (17), the edges of which are associated along prefracture lines (18, 19) respectively with said closure means (5) and with said neck (3).

8. The device according to one or more of the preceding claims, **characterized in that** said closure means (5) comprise a cylindrical sleeve (21), which is external and substantially coaxial to said tubular element (6) and is associated, so that it can slide axially and substantially hermetically, with said neck (3), at least one tab (22) for connecting said cylindrical sleeve (21) to said tubular element (6) as a single body, and a stopper (24), which is associated with the end of said body that lies opposite said container and is provided with a first cylindrical appendage (25) for forming a seal with said sleeve and with a second cylindrical appendage (26) for forming a seal with said tubular element (6), which is open at its opposite ends for the passage of said dispenser (12).

9. The device according to claim 8, **characterized in that** said closure means (5) comprise a ring (27) for locking said stopper (24) to said cylindrical sleeve (21).

10. The device according to one or more of the preceding claims, as dependent on claim 5, **characterized in that** said cap (15) is associated along prefracture lines with said closure means (5).

11. The device according to one or more of the preceding claims, **characterized in that** said sealing means (7), when associated with said tubular element (6), comprise an annular expansion (9) that protrudes from the outer lateral surface of said tubular element (6).

12. The device according to one or more of the preceding claims, as dependent on claims 3 and 8, **characterized in that** said first stop means (13) comprise a collar (29) that protrudes from the outer lateral surface of said sleeve (21) and a corresponding first annular abutment surface formed in said neck (3).

13. The device according to one or more of the preceding claims, as dependent on claim 3, **characterized in that** said second stop means (14) comprise an annular protrusion (32), which is formed on the outer lateral surface of said dispenser (12) and is suitable to interfere with a corresponding second annular abutment surface formed proximate to the end (6b) of said tubular element (6) that is open toward the outside.

14. The device according to one or more of the preceding claims, **characterized in that** said return means (11) comprise elastic means, such as springs (31) or fins that cooperate with a conical surface.

15. The device according to one or more of the preceding claims, **characterized in that** it comprises a valve (33), which is associated with said inlet end (12a) of the dispenser (12) and is suitable to prevent the escape of said first component (A) or of the mixture of said first (A) and second components (B) from said container (2) when said dispenser (12) is in the substantially retracted configuration.

16. The device according to one or more of the preceding claims, as dependent on claim 8, **characterized in that** said dispenser (12) comprises a contoured head, which is formed at said outlet end (12b) thereof and has an outside diameter that is substantially lager than the inside diameter of said tubular element (6) or of said second cylindrical appendage (26).

17. The device according to one or more of the preceding claims, **characterized in that** a portion of the walls of said container (2) formed at the base of said neck (3) is of the elastically deformable type.

18. The device according to one or more of the preceding claims, **characterized in that** said portion of the elastically deformable type is concertina shaped, with a compression axis that is substantially parallel to the longitudinal axis of said container (2).

## Patentansprüche

1. Vorrichtung (1) zur Speicherung und Ausgabe von zweikomponentigen Produkten, insbesondere für Scheiden-oder Rektalspülungen, die aufweist:
einen Behälter (2) einer ersten Komponente (A), der mit einem Hals (3) versehen ist, an dessen oberen Ende sich eine Öffnung (4) befindet;
eine Schließeinrichtung (5) für die Öffnung (4), die zusammen mit dem Hals (3) im Wesentlichen hermetisch dicht und in einer Weise, dass sie axial gleiten kann, verbunden ist und ein rohrförmiges Element (6), das an seinen entgegengesetzten Enden offen ist und im Inneren des Halses (3) verläuft und im Wesentlichen zu ihm koaxial ist, starr trägt;
eine Abdichteinrichtung (7) zum Bereitstellen einer Dichtung zusammen mit mindestens einem Teil (8) der Wände des Halses (3), die mit dem rohrförmigen Element (6) oder mit der Schließeinrichtung (5) verbunden ist;
eine rohrförmige Ausgabevorrichtung (12), die vom rohrförmigen Element (6) aufgenommen wird und in der das eintrittseitige Ende (12a) in Richtung des Innenraums des Behälters (2) gerichtet ist und das austrittseitige Ende (12b) außerhalb des rohrförmigen Elements (6) verläuft; und
eine temporäre Hemmeinrichtung (10) zum temporären Hemmen des Gleitens der Schließeinrichtung (5); **dadurch gekennzeichnet, dass**
das Volumen, das von der Innenfläche des Halses (3), von der Schließeinrichtung (5), von der äußeren seitlichen Fläche des rohrförmigen Elements (6) und von der Abdichteinrichtung (7) begrenzt wird, einen Tank (S) für eine zweite Komponente (B) bildet, und
nach dem Beseitigen der temporären Hemmeinrichtung (10) die Schließeinrichtung (5) zwischen einer ersten Konfiguration, in der die Abdichteinrichtung (7) in den Teil (8) der Wände des Halses (3) eingreift, wodurch der Tank (S) geschlossen wird, und einer zweiten Konfiguration, in der die Abdichteinrichtung (7) von dem Teil gelöst wird, wodurch der Tank (S) geöffnet wird, um die zweite Komponente (B) in den Behälter (2) zu gießen, gleitfähig ist.

2. Vorrichtung gemäß Anspruch 1, **dadurch gekennzeichnet, dass** die Ausgabevorrichtung (12) vom rohrförmigen Element (6) so aufgenommen wird, dass sie im Wesentlichen koaxial ist und, im Wesentlichen hermetisch dicht zusammen mit mindestens einem Teil ihrer seitlichen inneren Fläche, zwischen einer Konfiguration, in der sie im Wesentlichen in Richtung der Innenseite des Behälters (2) zurückgezogen ist, und einer Konfiguration, in der sie im Wesentlichen in Richtung der Außenseite des Behälters (2) ausgestreckt ist, in Bezug auf sie axial gleiten kann.

3. Vorrichtung gemäß einem oder mehr der vorangegangenen Ansprüche, **dadurch gekennzeichnet, dass** sie eine erste (13) und/oder eine zweite Einrichtung (14) zum Stoppen des Gleitens jeweils der Schließeinrichtung (5) und/oder der Ausgabevorrichtung (12) aufweist.

4. Vorrichtung gemäß einem oder mehr der vorangegangenen Ansprüche, **dadurch gekennzeichnet, dass** sie eine Rückstelleinrichtung (11) zum Zurückstellen der Schließeinrichtung (5) in die erste Konfiguration aufweist.

5. Vorrichtung gemäß einem oder mehr der vorangegangenen Ansprüche, **dadurch gekennzeichnet, dass** sie eine entfernbare Kappe (15) zum Abdecken des austrittsseitigen Endes (12b) der Ausgabevorrichtung (12) aufweist.

6. Vorrichtung gemäß einem oder mehr der vorangegangenen Ansprüche, dadurch gekennzeichet, dass sie eine entfernbare Abdichteinrichtung (16) aufweist, die zwischen der Schließeinrichtung (5) und dem Hals (3) gebildet wird.

7. Vorrichtung gemäß Anspruch 6, **dadurch gekennzeichnet, dass** die temporäre Hemmeinrichtung (10) und die entfernbare Abdichteinrichtung (16) mit einem ringförmigen Band (17) zusammenfallen und dieses aufweisen, dessen Kanten entlang von Bruchlinien (18, 19) mit der Schließeinrichtung (5) bzw. mit dem Hals (3) verbunden sind.

8. Vorrichtung gemäß einem oder mehr der vorangegangenen Ansprüche, **dadurch gekennzeichnet, dass** die Schließeinrichtung (5) aufweist eine zylindrische Manschette (21), die sich außerhalb des rohrförmigen Elements (6) befindet und im Wesentlichen koaxial mit diesem ist und mit dem Hals (3) so verbunden ist, dass sie axial und im Wesentlichen hermetisch dicht gleiten kann, mindestens einen Vorsprung (22) zum Verbinden der zylindrischen Manschette (21) mit dem rohrförmigen Element (6) zu einem einzigen Körper und einen Anschlag (24), der mit dem Ende des Körpers, das gegenüber dem Behälter liegt, verbunden ist und mit einem ersten zylindrischen Anhang (25) zum Bilden einer Abdichtung mit der Manschette und mit einem zweiten zylindrischen Anhang (26) zum Bilden einer Abdichtung mit dem rohrförmigen Element (6), das an seinen gegenüberliegenden Enden für den Durchgang der Ausgabevorrichtung (12) offen ist, versehen ist.

9. Vorrichtung gemäß Anspruch 8, **dadurch gekennzeichnet, dass** die Schließeinrichtung (5) einen Ring (27) zum Arretieren des Anschlages (24) an der zylindrischen Manschette (21) aufweist.

10. Vorrichtung gemäß einem oder mehr der vorangegangenen Ansprüche als Unteranspruch von Anspruch 5, **dadurch gekennzeichnet, dass** die Kappe (15) entlang von Bruchlinien mit der Schließeinrichtung (5) verbunden ist.

11. Vorrichtung gemäß einem oder mehr der vorangegangenen Ansprüche, **dadurch gekennzeichnet, dass** die Abdichteinrichtung (7), wenn sie mit dem rohrförmigen Element (6) verbunden ist, eine ringförmige Erweiterung (9) aufweist, die von der äußeren seitlichen Fläche des rohrförmigen Elements (6) vorspringt.

12. Vorrichtung gemäß einem oder mehr der vorangegangenen Ansprüche als Unteranspruch der Ansprüche 3 und 8, **dadurch gekennzeichnet, dass** die erste Stoppeinrichtung (13) einen Kragen (29), der von der äußeren seitlichen Fläche der Manschette (21) vorspringt, und eine entsprechende, im Hals (3) gebildete erste ringförmige Anstoßfläche aufweist.

13. Vorrichtung gemäß einem oder mehr der vorangegangenen Ansprüche als Unteranspruch von Anspruch 3, **dadurch gekennzeichnet, dass** die zweite Stoppeinrichtung (14) einen ringförmigen Vorsprung (32) aufweist, der auf der äußeren seitlichen Fläche der Ausgabevorrichtung (12) gebildet wird und geeignet ist, in eine entsprechende, zweite ringförmige Anstoßfläche einzugreifen, die nahe dem Ende (6b) des rohrförmigen Elements (6), das nach außen hin offen ist, gebildet wird.

14. Vorrichtung gemäß einem oder mehr der vorangegangenen Ansprüche, **dadurch gekennzeichnet, dass** die Rückstelleinrichtung (11) elastische Einrichtungen wie etwa Federn (31) oder Rippen, die mit einer konischen Fläche zusammenwirken, aufweist.

15. Vorrichtung gemäß einem oder mehr der vorangegangenen Ansprüche, **dadurch gekennzeichnet, dass** sie ein Ventil (33) aufweist, das mit dem eintrittsseitigen Ende (12a) der Ausgabevorrichtung (12) verbunden ist und geeignet ist, das Austreten der ersten Komponente (A) oder der Mischung aus der ersten (A) und der zweiten (B) Komponente aus dem Behälter (2) zu verhindern, wenn die Ausgabevorrichtung (12) sich in der im Wesentlichen zurückgezogenen Konfiguration befindet.

16. Vorrichtung gemäß einem oder mehr der vorangegangenen Ansprüche als Unteranspruch von Anspruch 8, **dadurch gekennzeichnet, dass** die Ausgabevorrichtung (12) einen profilierten Kopf aufweist, der an ihrem austrittsseitigen Ende (12b) gebildet wird und einen Außendurchmesser hat, der im Wesentlichen größer als der Innendurchmesser des rohrförmigen Elements (6) oder des zweiten zylindrischen Anhang (26) ist.

17. Vorrichtung gemäß einem oder mehr der vorangegangenen Ansprüche, **dadurch gekennzeichnet, dass** ein Teil der Wände des Behälters (2), die an der Basis des Halses (3) gebildet werden, von elastisch verformbarer Art ist.

18. Vorrichtung gemäß einem oder mehr der vorangegangenen Ansprüche, **dadurch gekennzeichnet, dass** der Teil von elastisch verformbarer Art die Form eines Faltenbalges hat, mit einer Pressachse, die im Wesentlichen parallel zur Längsachse des Behälters (2) verläuft.

## Revendications

1. Dispositif destiné à contenir et à distribuer des produits à deux composants, en particulier des solution de lavage vaginal ou rectal, comprenant :
un contenant (2) pour un premier composant (A), qui est pourvu d'un goulot (3) au sommet duquel se trouve une ouverture (4) ;
des moyens de fermeture (5) pour ladite ouverture (4), qui sont associés audit goulot (3), de manière essentiellement hermétique et de manière à pouvoir coulisser axialement, et supportent rigidement un élément tubulaire (6) qui est ouvert à ses extrémités opposées et s'étend à l'intérieur dudit goulot (3) et est essentiellement coaxial à celui-ci ;
des moyens d'étanchéité (7) destinés à former un joint d'étanchéité avec au moins une partie (8) des parois dudit goulot (3), qui sont associés audit élément tubulaire (6) ou auxdits moyens de fermeture (5) ;
un distributeur tubulaire (12) qui est reçu dans ledit élément tubulaire (6) et dans lequel l'extrémité d'entrée (12a) est orientée vers l'intérieur dudit contenant (2) et l'extrémité de sortie (12b) s'étend à l'extérieur dudit élément tubulaire (6) ; et
des moyens de verrouillage temporaire (10) pour verrouiller temporairement le coulissement desdits moyens de fermeture (5) ;
***caractérisé en ce que***
le volume délimité par la surface interne dudit goulot (3), par lesdits moyens de fermeture (5), par la surface latérale extérieure dudit élément tubulaire (6), et par lesdits moyens d'étanchéité (7) forment un réservoir (S) pour un deuxième composant (B), et
à la suite de l'élimination desdits moyens de verrouillage temporaire (10), lesdits moyens de fermeture (5) sont aptes à coulisser entre une première configuration, dans laquelle les moyens d'étanchéité (7) interfèrent avec ladite portion (8) des parois dudit goulot (3), fermant ledit réservoir (S), et une deuxième configuration, dans laquelle les moyens d'étanchéité (7) sont désengagés de ladite portion, ouvrant ledit réservoir (S) de manière à verser ledit deuxième composant (B) dans ledit récipient (2).

2. Dispositif selon la revendication 1, ***caractérisé en ce que*** ledit distributeur (12) est reçu dans ledit élément tubulaire (6) de façon qu'il soit sensiblement coaxial et puisse coulisser axialement par rapport à lui, de manière essentiellement hermétique avec au moins une partie de sa surface latérale intérieure, entre une configuration dans laquelle il est essentiellement rétracté vers l'intérieur dudit contenant (2) et une configuration dans laquelle il déborde essentiellement vers l'extérieur dudit contenant (2).

3. Dispositif selon l'une ou plusieurs des revendications précédentes, ***caractérisé en ce* qu'**il comprend des premiers (13) et/ou des deuxièmes (14) moyens pour arrêter le coulissement respectivement desdits moyens de fermeture (5) et/ou dudit distributeur (12).

4. Dispositif selon l'une ou plusieurs des revendications précédentes, ***caractérisé en ce* qu'**il comprend des moyens de renvoi (11) pour renvoyer lesdits moyens de fermeture (5) dans ladite première configuration.

5. Dispositif selon l'une ou plusieurs des revendications précédentes, ***caractérisé en ce* qu'**il comprend un bouchon amovible (15) pour couvrir ladite extrémité de sortie (12b) du distributeur (12).

6. Dispositif selon l'une ou plusieurs des revendications précédentes, ***caractérisé en ce* qu'**il comprend des moyens d'étanchéité amovibles (16) formés entre lesdits moyens de fermeture (5) et ledit goulot (3).

7. Dispositif selon la revendication 6, ***caractérisé en ce que*** lesdits moyens de verrouillage temporaire (10) et lesdits moyens d'étanchéité amovibles (16) coïncident avec, et comprennent, une bande annulaire (17) dont les bords sont associés, le long de lignes prédécoupées (18, 19), respectivement avec lesdits moyens de fermeture (5) et avec ledit goulot (3).

8. Dispositif selon l'une ou plusieurs des revendications précédentes, ***caractérisé en ce que*** lesdits moyens de fermeture (5) comprennent un manchon cylindrique (21), qui est extérieur et sensiblement coaxial audit élément tubulaire (6) et est associé, de manière à pouvoir coulisser axialement et de manière sensiblement hermétique, avec ledit goulot (3), au moins une languette (22) destinée à raccorder ledit manchon cylindrique (21) audit élément tubulaire (6) en un corps unique, et une butée (24), qui est associée à l'extrémité dudit corps qui est opposée audit contenant et est pourvue d'un premier appendice cylindrique (25) pour former un joint d'étanchéité avec ledit manchon, et d'un deuxième appendice cylindrique (26) pour former un joint d'étanchéité avec ledit élément tubulaire (6), qui est ouvert à ses extrémités opposées pour le passage dudit distributeur (12).

9. Dispositif selon la revendication 8, ***caractérisé en ce que*** lesdits moyens de fermeture (5) comprennent une bague (27) pour assujettir ladite butée (24) audit manchon cylindrique (21).

10. Dispositif selon l'une ou plusieurs des revendications précédentes, lorsqu'elles sont dépendantes de la revendication 5, ***caractérisé en ce que*** ledit bouchon (15) est associé, le long de lignes prédécoupées, auxdits moyens de fermeture (5).

11. Dispositif selon l'une ou plusieurs des revendications précédentes, ***caractérisé en ce que*** lesdits moyens d'étanchéité (7), lorsqu'ils sont associés audit élément tubulaire (6), comprennent un prolongement annulaire (9) qui fait saillie de la surface latérale extérieure dudit élément tubulaire (6).

12. Dispositif selon l'une ou plusieurs des revendications précédentes, lorsqu'elles sont dépendantes des revendications 3 et 8, ***caractérisé en ce que*** lesdits premiers moyens de butée (13) comprennent un collier (29) qui fait saillie de la surface latérale extérieure dudit manchon (21) et une première surface d'aboutement annulaire formée dans ledit goulot (3).

13. Dispositif selon l'une ou plusieurs des revendications précédentes, lorsqu'elles sont dépendantes de la revendication 3, ***caractérisé en ce que*** lesdits deuxièmes moyens de butée (14) comprennent une saillie annulaire (32) qui est formée sur la surface latérale extérieure dudit distributeur (12) et est apte à interférer avec une deuxième surface annulaire d'aboutement correspondante formée à proximité de l'extrémité (6b) dudit élément tubulaire (6) qui est ouverte vers l'extérieur.

14. Dispositif selon l'une ou plusieurs des revendications précédentes, ***caractérise en ce que*** lesdits moyens de renvoi (11) comprennent des moyens élastiques, tels que des ressorts (31) ou des ailettes qui coopèrent avec une surface conique.

15. Dispositif selon l'une ou plusieurs des revendications précédentes, ***caractérisé en* ce qu'**il comprend une valve (33) qui est associée ladite extrémité d'entrée (12a) du distributeur (12) et est apte à empêcher la fuite dudit premier composant (A) ou du mélange desdits premier (A) et deuxième (B) composants dudit contenant (2) lorsque ledit distributeur (12) est dans la configuration essentiellement rétractée.

16. Dispositif selon l'une ou plusieurs des revendications précédentes lorsqu'elles sont dépendantes de la revendication 8, ***caractérisé en ce que*** ledit distributeur (12) comprend une tête profilée, qui est formée au niveau de ladite extrémité de sortie (12b) de celui-ci et possède un diamètre extérieur qui est sensiblement supérieur au diamètre intérieur dudit élément tubulaire (6) ou dudit deuxième appendice cylindrique (26).

17. Dispositif selon l'une ou plusieurs des revendications précédentes, ***caractérisé en ce* qu'**une portion des parois dudit contenant (2) formée à la base du goulot (3) est du type élastiquement déformable.

18. Dispositif selon l'une ou plusieurs des revendications précédentes, ***caractérisé en ce que*** ladite portion du type élastiquement déformable est de type en accordéon, avec un axe de compression qui est sensiblement parallèle à l'axe longitudinal dudit contenant (2).
